Europäisches Patentamt

European Patent Office

Office européen des brevets

(19)

(11) Publication number: **0 079 615**
**B1**

(12) # EUROPEAN PATENT SPECIFICATION

(45) Date of publication of patent specification: **16.04.86**

(51) Int. Cl.⁴: **A 61 N 5/00, H 03 K 17/96**

(21) Application number: **82110590.5**

(22) Date of filing: **16.11.82**

(54) **Apparatus for laser light medical treatment.**

(30) Priority: **18.11.81 JP 185060/81**
**18.11.81 JP 185061/81**

(43) Date of publication of application:
**25.05.83 Bulletin 83/21**

(45) Publication of the grant of the patent:
**16.04.86 Bulletin 86/16**

(84) Designated Contracting States:
**DE FR GB IT**

(56) References cited:
**DE-A-2 548 354**
**DE-A-2 550 327**
**FR-A-2 420 352**
**GB-A-1 022 722**
**GB-A-2 064 104**
**US-A-3 622 743**
**US-A-3 727 101**
**US-A-3 944 843**
**US-A-4 232 678**

(73) Proprietor: **Matsushita Electric Industrial Co., Ltd.**
**1006, Oaza Kadoma**
**Kadoma-shi Osaka-fu, 571 (JP)**

(73) Proprietor: **Japan Medical Laser Laboratory Co., Ltd.**
**7, Koujimachi 5-chome Chiyoda-ku Tokyo-to (JP)**

(72) Inventor: **Kubo, Kiyoshi**
**11-1, Myoken-higashi 4-chome**
**Katano City, 576 (JP)**
Inventor: **Ohshiro, Toshio**
**13-8, Higashi-nakano 4-chome**
**Nakano-ku Tokyo-to, 164 (JP)**

(74) Representative: **Patentanwälte Kirschner & Grosse**
**Herzog-Wilhelm-Strasse 17**
**D-8000 München 2 (DE)**

Courier Press, Leamington Spa, England.

## Description

### Background of the Invention

### 1. *Field of the Invention*

The present invention relates to an improvement in an apparatus for laser beam treatment. More particularly the present invention relates to an improvements to avoid possible danger to human eyes by invisible laser beam in an apparatus for laser beam treatment.

Recently, many studies of medical treatments such as acupuncture or the like are made. And among them, treatment of various aches such as toothache, lumbago, stiffness in the shoulders arms and legs, are made by use of laser light beam. And recently, relations and effect of intensity, treating time, wavelength of light and repetition period of light beam pulses on such medical treatment are more and more made clear with respect to therapeutic effect. However, causality of photochemical effect of the laser beam on medical treatment has some unclear points. Many reports are made on application of laser beam on medical treatment. But those on operativity and safetyness of the apparatus of such medical treatment is not yet enough.

DE—A—25 48 354 discloses a laser light medical apparatus as defined in the first part of claim 1, in which a laser light beam is emitted through an emission hole of a hand set to the skin of a patient. Close to the emission hole sensing means comprising a pair of electrodes is provided and switching means cut off the emission of said laser light beam in response to a signal provided by said sensing means if the resistance of the skin exceeds predetermined upper or lower values in order to detect correct acupuncture points.

The laser light beam, different from usual light beam, is of coherent light and can be easily focused. Accordingly, even with using such a rather small laser light beam of for instance, several milli watt, it is possible to obtain a very high power density of the light beam by effectively converging the light beam into a very small area, for instance, several to fractional $mm^2$. In medical treatment, usually a laser light in a wavelength range, for instance of infrared light range, light of which is easily absorbed in human skin, is generally used. Accordingly, when such invisible laser light erroneously shoots human eyes by a maloperation, then retina of eye may be easily destroyed to make the patient blind.

### Summary of the Invention

Therefore, the purpose of the present invention is to provide an improved apparatus for laser beam treatment which has more safety by providing some safety measures. Also, according to a particular embodiment of the invention, operation of the laser light emission is warned by warning sound or/and warning light. By such safety measures, inadvertent shooting the human eye by the laser light beam can be avoided, and further, operation for an appropriate time length of treatment is easy.

### Brief Explanation of the Drawing

FIG. 1 is a perspective view of one example embodying the present invention.

FIG. 2 is a circuit block diagram of the apparatus of FIG. 1.

FIG. 3 is a perspective view of a hand set of the apparatus of FIG. 1.

FIG. 4 is a front view of the hand set of FIG. 3.

FIG. 5 is a partial sectional view of the hand set of FIG. 3.

FIG. 6 is a detailed circuit diagram of a protection circuit 61 of the circuit of FIG. 2.

FIG. 7 is a perspective view of another example embodying the present invention.

FIG. 8 is a circuit diagram of the apparatus of FIG. 7.

### Description of the Preferred Embodiments

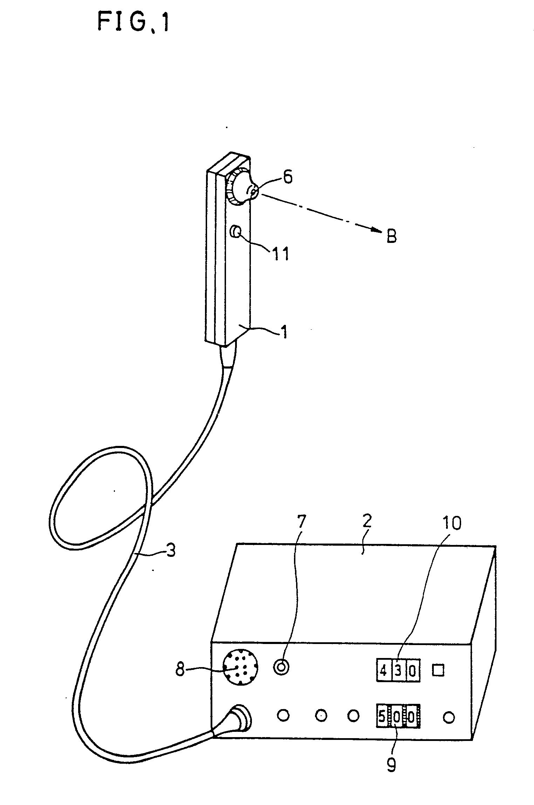

FIG. 1 shows general view of the apparatus for laser beam medical treatment in accordance with the present invention. The apparatus comprises a hand set 1 and a circuit part 2 and an electric cable 3 connecting the above-mentioned two parts. The hand set 1 has a laser light emitting hole 6 which is to be applied to a local point of patient for treatment. The hand set 1 further has a switch 11 for controlling on and off of the laser light emission. The circuit part 2 has a time setting switch 9 for setting a time length for treatment and a numeral indicator 10 for indicating a set time and subsequent remaining time during treatment. The circuit part 2 further has a warning lamp 7 and small speaker 8 which are for indicating that laser light beam is being emitted.

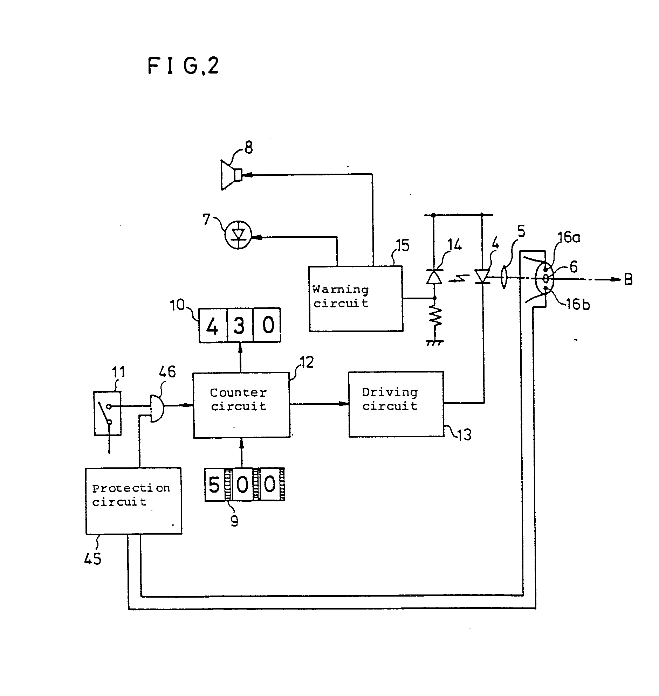

FIG. 2 diagrammatically shows connection of the apparatus of FIG. 1, wherein control switch 11 gives its outputs through an AND gate 46, a counter circuit 12 and a driving circuit 13 which feeds laser operation current to a laser 4 which emit light beam through a lens 5 and through emission hole 6. The time setting switch 9 gives time set output to the counter 12 and the latter gives output to the numeral indicator 10. A phototransistor 14 is provided to receive a back light of a laser and gives its output to a warning circuit 15, which gives outputs to the warning lamp 7 and the small speaker 8.

The operation for medical treatment of the FIG. 2, circuit is elucidated hereafter. At first, the user sets the time-set switch 9 for example, 5 min and 0 sec, then the set time is given to the counter 12 and the set time is indicated on the numeral indicator 10. Then by pushing the control switch 11 on the hand set 1, the control signal is given to an AND gate, which at receipt of a signal from the protection circuit 45 gives signal through the counter circuit 12, the driving circuit 13, and actuate the laser to emits laser light beam through the emission hole 6 of the hand set 1. As time lapses the numeral indicator 10 changes its indication thereby indicates remaining time. When the remaining time indication become zero, at this time the counter 12 stops to give the output, therefore the laser 4 stops emission of light. FIG. 2 shows that the numeral indicator shows 4 min and 30 sec remaining for treatment. During emission of the laser light from the emission hole 6 the warning lamp 7 or/and small speaker 8 issues warning light or/and warning sound so that user or the apparatus becomes careful. By means of the above numeral indicator 10 the user can accurately know the remaining time and avoid excessively long time application of the light and also feel easy by knowing the remaining time.

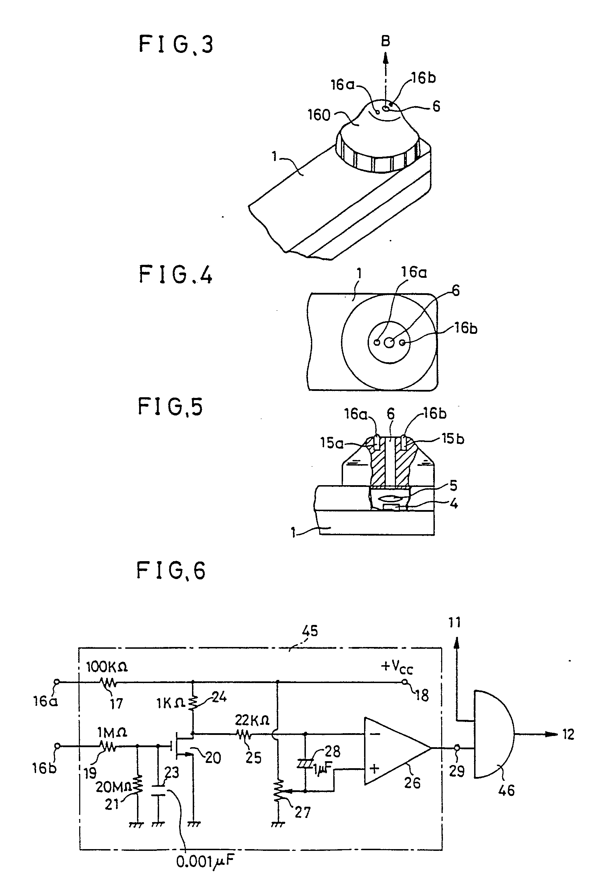

FIG. 3 shows detail around the light emission hole 6 of the hand set 1. On both sides of the light emission hole 6, there are provided a pair of contact tips 16a and 16b of sensor 15a and 15b, respectively as shown in FIG. 3, FIG. 4 and FIG. 5.

The contact sensor 15a and 15b are for detection of contacting or proximation of the light emission hole 6 to human skin. The sensor may be a capacitive switch or a sensor to detect minute current through electrode contacts to the human skin. In this example the sensor 15a and 15b are the electric conduction current detection means. When the contact tips 16a and 16b touch the human skin, the protection circuit 45 issues an output to the AND gate 46, thereby making the counter circuit 12 to count and gives output to the driving circuit 13, thereby to actuate the laser 4.

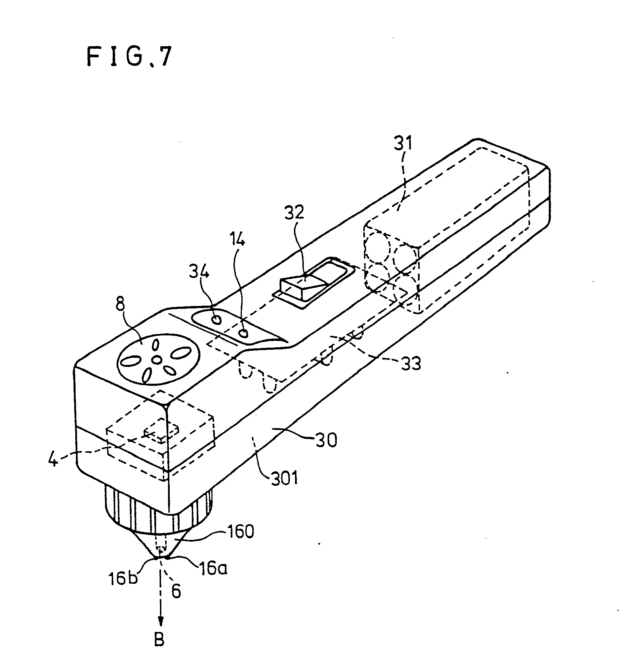

FIG. 6 shows one example of an actual circuit diagram of the protection circuit 45. The contact tip 16a is connected through a resistor 17 of 100 kΩ to the positive power source terminal 18. Another contact tip 16b is connected through a resistor 19 of 1 MΩ to a gate electrode of a MOS-FET 20. The gate electrode is grounded by a parallel connection of a resistor 21 of 20 MΩ and a capacitor 23 of 0.001 μF. The source of the MOS-FET 20 is grounded and the drain is connected through a resistor 24 of 1 kΩ to the positive power source terminal 18. The drain of the MOS-FET 20 is connected through a resistor 25 to a negative input terminal − of a differential amplifier 26. A potentiometer 27 is connected between the positive power source terminal 18 and the ground, and a sliding terminal of the potentiometer 27 is connected to a positive input terminal + of the differential amplifier 26. A capacitor 28 of a large capacitance for instance, 1 μF is connected across the negative input terminal − and the positive input terminal + of the differential amplifier 26. The output terminal 29 of the differential amplifier 26 is connected to an input terminal of an AND gate 46, output of which is to be given to the input terminal of the counter circuit 12. In the circuit of FIG. 6, the resistor 19 and capacitor 23 form a low pass filter, and the resistor 25 and the capacitor 28 form another low pass filter. These low pass filters serve to eliminate spurious noise based on commercial power line current when the contact tip 16b only touches the skin, thereby avoid maloperation.

When both contact tips 16a and 16b touch the human skin, then, though varying depending on the part of the body the resistance between the contact tips becomes from 100 kΩ to several hundred MΩ. That is the contact tips 16a and 16b are connected by a high resistance of the skin. Accordingly, a current flows from the power source terminal 18 through the resistor 17, contact tip 16a, skin contact tip 16b, the resistor 19 and the resistor 21 to the ground. Accordingly, a voltage is induced across the resistor 21 and the voltage is impressed on the gate electrode of the MOS-FET 20. Therefore, the MOS-FET 20 makes its drain current flow and the differential amplifier 26 amplifies change of its negative input terminal voltage, thereby issuing output to the AND gate circuit 62. Therefore, when both contacts 16a and 16b touch the skin, the signal from the protection circuit 45 is introduced to the AND gate 46 to actuate the counter circuit 12 and hence the laser 4, to emit the laser light.

Instead of the above-mentioned proximity detection way by utilizing contact current conduction on skin, another way of proximity detection, for instance by use of known capacitance change detection by proximity of electrode to the human skin may be used similarly.

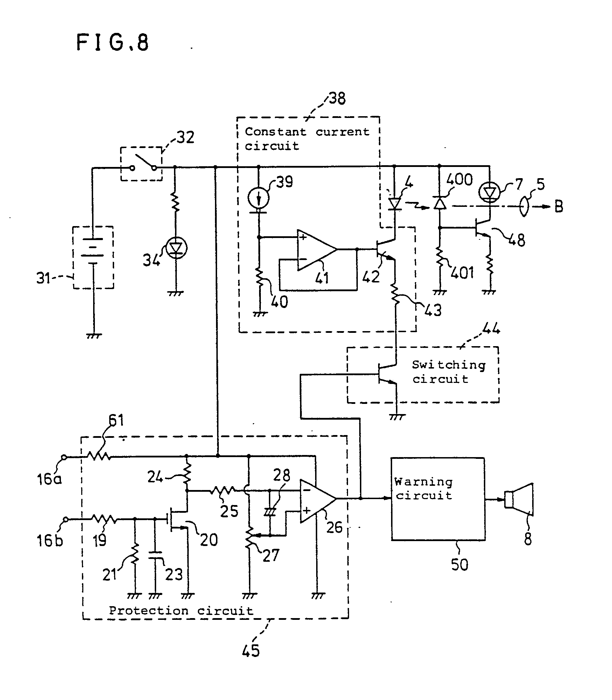

FIG. 7 and FIG. 8 show another example of an apparatus for laser beam medical treatment of the present invention wherein FIG. 7 is a perspective general view of the apparatus, and FIG. 8 shows a circuit diagram of the apparatus. Corresponding parts and components to that of the first example are designated by the same numerals. This apparatus is characterized by combining a hand set and a circuit part in a single integral unit casing 30. The casing 30 contains one or a set of batteries 31 as power source mean in its grip part. By turning on of a power switch 32, the power is fed from the battery 31 to the circuit. The casing also contains a power switch indication lamp 34, which indicates that the power source circuit is ON. The casing 30 further contains a small speaker 8 and warning lamps 7 for warning that the laser 4 is emitting light. On the front face 301, a laser emitting mound 160 is provided whereon the light emission hole 6 is provided and the pair of contact tips 16a and 16b on sensors are provided. The casing further contains printed circuit board 33 having a circuit shown in FIG. 8.

In the circuit of FIG. 8, the protection circuit 45 is substantially the same as that of the circuit of FIG. 6 and the output of the circuit 61 is given through a switching circuit 44 to a terminal of the laser 4 so as to control ON-OFF of the laser 4, and the output is also given to a warning signal generator for actuating the small speaker 8.

The operation of FIG. 8 is elucidated. When a power switch 32 is closed, current from the power source 31 is fed to the circuit and the pilot lamp 34 for showing the switching-on is lit. When the contact tips 16a and 16b touch the skin, then output signal is issued from the differential amplifier 26 to the warning circuit 46 and to the switching circuit 44.

The switching circuit 44 controls the ON-OFF of the laser 4, and the laser current is controlled constant by a constant current circuit 38 comprising a transistor 42, an operational amplifier 41 and a constant voltage circuit consisting of a constant voltage device 39 and a resistor 40. A part of laser light, for instance back light of the laser is led to the phototransistor 400 which is connected in series with a resistor 401. The junction point between the phototransistor 400 and resistor 401 is connected to the base of a transistor 48, to the collector of which an LED 7 for indicating laser operation is connected. The laser 4 operates to emit a laser light beam of a constant intensity irrespective of wide variation of the voltage of the power source 31. And the warning lamp 7 warns operation of the laser. Also the warning circuit 50 issues warning signal to the small speaking 8. Accordingly, a warning sound is produced to warn the laser light beam is under emission. The warning circuit 50 is preferably a circuit to produce a comfortable music in order to ease the feeling of patient. Besides, an intermittent switching circuit such as monomultivibrator is preferably inserted in the base of the transistor 48 so as to intermittently lighting the LED 7.

The warning is necessary since the medical treating laser light is usually infrared light so that patient or user cannot recognize its emission.

The above-mentioned apparatus of FIG. 7 is very easy to handle since all the components and circuits are contained in the hand set casing compactly.

## Claims

1. An apparatus for laser light medical treatment comprising:

a hand set casing (1),

laser light beam emitting means (4) contained in said hand set casing and for applying a laser light beam through an emission hole (6) of the hand set casing to the skin of a patient for medical treatment,

sensing means provided close to said emission hole and comprising a pair of electrodes (16a, 16b) and switching means (45, 46) to cut off emission of said laser light beam in response to a signal provided by said sensing means,

characterized in that

said sensing means comprise a proximity sensor, said pair of electrodes (16a, 16b) being disposed on opposite sides of said emission hole (6) for electrical detection of the proximity of the skin to said electrodes, and said switching means cut off emission of said laser light beam when the proximity of said emission hole to a skin is not detected by said proximity sensor.

2. An apparatus for laser light medical treatment in accordance with claim 1, characterized in that said electrodes are for detection of the proximity by passing of a current thereacross through a skin touched by them.

3. An apparatus for laser light medical treatment in accordance with claim 1, characterized in that said electrodes are for detection of proximity by a change of capacitance thereof.

4. An apparatus for laser light medical treatment in accordance with claim 1, characterized by timer means for controlling said laser light beam emission in a manner to be emitted for a desired set time period.

5. An apparatus for laser light medical treatment in accordance with claim 1, characterized by time period indicating means for indicating remaining treating time period during the treating.

6. An apparatus for laser light medical treatment in accordance with claim 1, characterized by warning means for warning when the laser light beam emitting means is in an emitting state.

7. An apparatus for laser light medical treatment in accordance with claim 6, characterized in that said warning means comprises a photoelectric element which issues a signal when receiving a back light of said laser light beam emitting means.

8. An apparatus for laser light medical treatment in accordance with claim 7, characterized in that said warning means is sound producing means.

9. An apparatus for laser light medical treatment in accordance with claim 6 or 7, characterized in that said laser light emitting means emit invisible light, and said warning means is light emitting means which emit visible light.

10. An apparatus for laser light medical treatment in accordance with any of the preceding claims, characterized in that said hand set casing comprises a battery as a power source for said laser light beam emitting means and other electric circuit means.

11. An apparatus for laser light medical treatment in accordance with claim 10, characterized in that said hand set casing further comprises a manual power switch for manual controlling of current from said power source.

## Revendications

1. Appareil pour traitement médical par rayonnement laser comprenant:

— un boîtier portatif (1);

— un moyen (4) émetteur de faisceau de rayonnement laser contenant dans le boîtier portatif et destiné à appliquer un faisceau de rayonnement laser par l'intermédiaire d'un trou d'émission (6) ménagé dans le boîtier portatif à la peau d'un patient pour traitement médical;

— un moyen de détection prévu à proximité du trou d'émission et comportant une paire d'électrodes (16a, 16b) et

— un moyen de commutation (45, 46) pour couper l'émission du faisceau de rayonnement laser en réponse à un signal fourni par le moyen de détection, caractérisé en ce que:

— le moyen de détection comprend un capteur de proximité, la paire d'électrodes (16a, 16b) étant disposée sur les côtés opposés du trou d'émission (6) pour la détection électrique de la proximité de la peau vis-à-vis des électrodes, et le moyen de commutation coupe l'émission du faisceau de rayonnement laser lorsque la proximité du trou d'émission vis-à-vis de la peau n'est pas détectée par le capteur de proximité.

2. Appareil pour traitement médical par rayonnement laser selon la revendication 1, caractérisé en ce que les électrodes servent à la détection de la proximité par passage d'un courant entre elles et à travers la peau avec laquelle elles sont en contact.

3. Appareil pour traitement médical par rayonnement laser selon la revendication 1, caractérisé en ce que les électrodes servent à la détection de la proximité par variation de leur capacité.

4. Appareil pour traitement médical par rayonnement laser selon la revendication 1, caractérisé par un moyen de minuterie pour contrôler l'émission du faisceau de rayonnement de façon à ce que celui-ci soit émis pendant une durée préétablie désirée.

5. Appareil pour traitement médical par rayonnement laser selon la revendication 1, caractérisé par un moyen indicateur de durée destiné à indiquer le temps restant du traitement au cours du traitement.

6. Appareil pour traitement médical par rayonnement laser selon la revendication 1, caractérisé par un moyen avertisseur destiné à donner un avertissement lorsque le moyen émetteur de faisceau de rayonnement laser se trouve à l'état d'émission.

7. Appareil pour traitement médical par rayonnement laser selon la revendication, 6, caractérisé en ce que le moyen avertisseur comprend un élément photo-électrique qui émet un signal lors de la réception d'une lumière de renvoi provenant du moyen émetteur de faisceau de rayonnement laser.

8. Appareil pour traitement médical par rayonnement laser selon la revendication 7, caractérisé en ce que le moyen avertisseur est un moyen produisant un son.

9. Appareil pour traitement médical par rayonnement laser selon la revendication 6 ou la revendication 7, caractérisé en ce que le moyen émetteur de rayonnement laser émet une lumière invisible, et en ce que le moyen avertisseur est un moyen émetteur de lumière qui émet une lumière visible.

10. Appareil pour traitement médical par rayonnement laser selon l'une quelconque des revendications précédentes, caractérisé en ce que le boîtier portatif comporte une pile comme source d'alimentation du moyen émetteur de faisceau de rayonnement laser et des autres moyens de circuits électriques.

11. Appareil pour le traitement médical par rayonnement laser selon la revendication 10, caractérisé en ce que le boîtier portatif comporte en outre un commutateur manuel d'alimentation pour la commande manuelle du courant provenant de la source d'alimentation.

**Patentansprüche**

1. Gerät zur medizinischen Laserlichtbehandlung enthaltend:
ein Handapparatgehäuse (1),
eine einen Laserlichtstrahl emittierende Einrichtung (4), die in dem Handapparatgehäuse enthalten und dazu bestimmt ist, einen Laserlichtstrahl durch ein Abstrahlungsloch (6) des Handapparatgehäuses auf die Haut eines Patienten zur medizinischen Behandlung zu applizieren,
eine Fühlereinrichtung, die nahe bei dem Abstrahlungsloch angeordnet ist und zwei Elektroden (16a, 16b) sowie eine Schalteinrichtung (45, 46) aufweist, um die Abstrahlung des Laserlichtstrahls in Abhängigkeit von einem von der Fühlereinrichtung gelieferten Signal abzuschalten,
dadurch gekennzeichnet,
daß die Fühlereinrichtung einen Nahwirkungsfühler aufweist, wobei die zwei Elektroden (16a, 16b) an gegenüberliegenden Seiten des Abstrahlungslochs (6) angeordnet sind, um auf elektrischem Wege das Indernäheliegen der Haut bei den Elektroden festzustellen, und wobei die Schalteinrichtung die Abstrahlung des Laserlichtstrahles abschaltet, wenn das Indernäheliegen des Abstrahlungsloches an der Haut von dem Nahwirkungsfühler nicht festgestellt wird.

2. Gerät zur medizinischen Laserlichtbehandlung nach Anspruch 1, dadurch gekennzeichnet, daß die Elektroden dazu bestimmt sind, das Indernäheliegen dadurch festzustellen, daß ein Strom durch die von ihnen berührte Haut zwischen ihnen fließt.

3. Gerät zur medizinischen Laserlichtbehandlung nach Anspruch 1, dadurch gekennzeichnet, daß die Elektroden dazu bestimmt sind, das Indernäheliegen durch eine Änderung ihrer Kapazität festzustellen.

4. Gerät zur medizinischen Laserlichtbehandlung nach Anspruch 1, gekennzeichnet durch eine Zeitgebereinrichtung, um das Abstrahlen des Laserlichtstrahles in der Weise zu steuern, daß er während einer gewünschten, vorgegebenen Zeitdauer abgestrahlt wird.

5. Gerät zur medizinischen Laserlichtbehandlung nach Anspruch 1, gekennzeichnet durch eine Zeitdauer-Anzeigeeinrichtung, um während der Behandlung die verbleibende Behandlungszeitdauer anzuzeigen.

6. Gerät zur medizinischen Laserlichtbehandlung nach Anspruch 1, gekennzeichnet durch eine Warneinrichtung, um eine Warnung abzugeben, wenn die den Laserlichtstrahl emittierende Einrichtung in dem Abstrahlungs-Betriebszustand ist.

7. Gerät zur medizinischen Laserlichtbehandlung nach Anspruch 6, dadurch gekennzeichnet, daß die Warneinrichtung ein photoelektrisches Element aufweist, das ein Signal abgibt, wenn es reflektiertes Licht von der den Laserstrahl emittierenden Einrichtung erhält.

8. Gerät zur medizinischen Laserlichtbehandlung nach Anspruch 7, dadurch gekennzeichnet, daß die Warneinrichtung eine einen Schall erzeugende Einrichtung ist.

9. Gerät zur medizinischen Laserlichtbehandlung nach Anspruch 6 oder 7, dadurch gekennzeichnet, daß die den Laserstrahl emittierende

**0 079 615**

Einrichtung nicht-sichtbares Licht abstrahlt, und daß die Warneinrichtung eine Licht emittierende Einrichtung ist, die sichtbares Licht abstrahlt.

10. Gerät zur medizinischen Laserlichtbehandlung nach einem der vorhergehenden Ansprüche, dadurch gekennzeichnet, daß das Handapparatgehäuse eine Batterie als Stromquelle für die das Laserlicht emittierende Einrichtung und weitere elektrische Schaltungseinrichtungen enthält.

11. Gerät zur medizinischen Laserlichtbehandlung nach Anspruch 10, dadurch gekennzeichnet, daß das Handapparatgehäuse ferner einen Handstromschalter umfaßt, um den Strom von der Stromquelle von Hand zu steuern.

0 079 615

FIG.1

F I G.2

FIG.3

FIG.4

FIG.5

FIG.6

# FIG.7

# FIG.8